# EUROPEAN PATENT SPECIFICATION

(11) **EP 3 531 367 B1**
(45) Date of publication and mention of the grant of the patent: **20.01.2021**
(21) Application number: 16919039.4
(22) Date of filing: 25.11.2016
(51) Int. Cl.: A01G 23/00

(54) **SYSTEM FOR PREDICTING OCCURRENCE OF PINE WILT DISEASE AND METHOD THEREFOR**
SYSTEM ZUR VORHERSAGE DES AUFTRETENS VON KIEFERERKRANKUNGEN UND VERFAHREN DAFÜR
SYSTÈME DE PRÉDICTION DE L'APPARITION D'UNE MALADIE DES PINS ET PROCÉDÉ ASSOCIÉ

(30) Priority: 21.10.2016 KR 20160137856
(43) Date of publication of application: 28.08.2019
(73) Proprietor: Econnbiz Co., Ltd., Sejong 30023 (KR)
(72) Inventor: PARK, Seong Bean, Suwon-si, Gyeonggi-do 16376 (KR); PARK, Yong Chan, Goyang-si, Gyeonggi-do 10214 (KR)
(74) Representative: M. Zardi & Co S.A.
(86) International application number: PCT/KR2016/013689
(87) International publication number: WO 2018/074655

(56) References cited:
- CN-U- 205 373 752
- JP-B2- 4 600 884
- KR-A- 20120 093 390
- KR-A- 20130 003 143
- KR-A- 20160 107 806
- KR-B1- 101 548 587
- KR-B1- 101 548 587
- WOO KWAN-SOO ET AL: "Early Disease Development and Stem and Leaf Water Content in the Seedlings of Pinus koraiensis Inoculated with Pinewood Nematodes in a Greenhouse", PLANT PATHOLOGY JOURNAL, HAN'GUG SIGMUL BYEONGRI HAGHOE, KP, vol. 25, no. 3, 30 September 2009 (2009-09-30), pages 241-246, XP009516101, ISSN: 0256-8608, DOI: 10.5423/PPJ.2009.25.3.241

## Description

### BACKGROUND

Embodiments of the inventive concept described herein relate to a system and a method for predicting occurrence of a tree disease, and more particularly, relate to a system and a method for predicting whether a tree disease is to occur using moisture information about a tree and soil or sugar content information about the tree.

There are many types of a tree disease, and a representative example of thereof is a pine wilt disease. The pine wilt disease is a disease in which a pine is dried to be killed caused by a pinewood nematode parasitic on a Monochamus alternatus. When the pine is infected with the pine wilt disease, the pine is 100 % killed. Therefore, a damage is increasing every year since the pine wilt disease was first recognized in 1988 at Geumj eong Mountain in Busan.

However, in a case of the tree disease, it is difficult to visually observe a pre-symptom. In a case of the pine wilt disease, which the representative tree disease, a direction of movement of the Monochamus alternatus that moves the pinewood nematode and characteristics of spread of the pine wilt disease have not been revealed to date.

Therefore, prevention activities has been carried out to prevent the tree disease. However, an effect of the prevention activity is insignificant, and there is no specific way to fundamentally block onset of the tree disease. Accordingly, in order to find and block a diffusion path of the insect vector, periodic aerial/land-based chemical spraying, control methods such as lumbering an infection tree, fumigating, and the like for removing the pinewood nematode and an insect vector at the same time, and the like are only used.

Thus, as of now, it is best to take a quick action after checking whether the tree disease has occurred. To this end, the present inventors have devised the inventive concept that uses various sensors and analyzes data from the sensors to predict the tree disease and prevent the spread of the tree disease. KR 10/1548587 B1 discloses a measuring instrument for diagnosing trees and a method for measuring a vital degree of trees using the same. In terms of the measuring instrument for diagnosing trees, a sensor for the vital degree of trees and a soil information measuring sensor are connected to a measuring body, and an atmospheric information measuring sensor and a location information sensor are provided inside the measuring body.

### SUMMARY

Conventional patents are generally designed for predicting a pine wilt disease which is a representative tree disease. In main methods of the conventional patents, whether flow of sap in a tree is smooth or not is detected to judge whether the pine wilt disease has occurred. However, withering of the tree is not merely because a water stream of the tree is blocked. Therefore, in order to improve a prediction accuracy of the pine wilt disease, it is necessary to consider other factors besides whether the flow of the sap in the tree is smooth.

In addition, in order to predicting the pine wilt disease using various sensors, information sensed by the sensors must be transmitted to a server. However, conventionally, in an existing communication method, not only a reach of a radio wave is short, but also a sensor for collecting data consumes power. Thus, utilizing a sensor itself, which is to be used in prior arts, was not possible in a forest that is far from a radio base station and where power supply is not good.

The above technical problem is solved by the system for predicting occurrence of the pine wilt disease according to claim 1 and a method for predicting whether a pine wilt disease is to occur according to claim 2.

Challenges to be solved by the inventive concept are not limited to those mentioned above, and other challenges not mentioned may be clearly understood by those skilled in the art from the description below.

### BRIEF DESCRIPTION OF THE FIGURES

The above and other objects and features will become apparent from the following description with reference to the following figures, wherein like reference numerals refer to like parts throughout the various figures unless otherwise specified, and wherein:
FIG. 1 is a block diagram of a system for predicting occurrence of a tree disease according to an example explaining the background of the invention;
FIG. 2 is an expanded block diagram of a system for predicting occurrence of a tree disease according to an example explaining the background of the invention;
FIG. 3 is a block diagram of a tree disease-occurrence prediction device according to an example explaining the background of the invention;
FIG. 4 is a block diagram of a tree disease-occurrence prediction device according to an example explaining the background of the invention;
FIG. 5 is a block diagram of a tree disease-occurrence prediction device according to an example explaining the background of the invention;
FIG. 6 is a block diagram of a tree disease-occurrence prediction device according to an example explaining the background of the invention;
FIG. 7 is a block diagram of a tree disease-occurrence prediction device according to an example explaining the background of the invention,
FIG. 8 is a block diagram of a tree disease-occurrence prediction device according to an example explaining the background of the invention;
FIG. 9 is a block diagram of a tree disease-occurrence prediction server according to an embodiment of the inventive concept; and
FIG. 10 is a flowchart of a method for predicting occurrence of a tree disease according to an embodiment of the inventive concept.

### DETAILED DESCRIPTION

The advantages and features of the inventive concept and a method for achieving the same will become apparent from the following description of the following embodiments given in conjunction with the accompanying drawings. The same reference numerals denote the same elements throughout the specification.

Unless otherwise defined, all terms (including technical and scientific terms) used herein have the same meaning as commonly understood by those skilled in the art to which the inventive concept pertains. It will be further understood that terms, such as those defined in commonly used dictionaries, should not be interpreted in an idealized or overly formal sense unless expressly so defined herein.

The terms used herein are provided to describe the embodiments but not to limit the inventive concept. In the specification, the singular forms include plural forms unless particularly mentioned. The terms "comprises" and/or "comprising" used herein does not exclude presence or addition of one or more other elements, in addition to the aforementioned elements.

As used herein, the term "unit" may have a hardware configuration, but a portion thereof may be a functional unit implemented in a software manner.

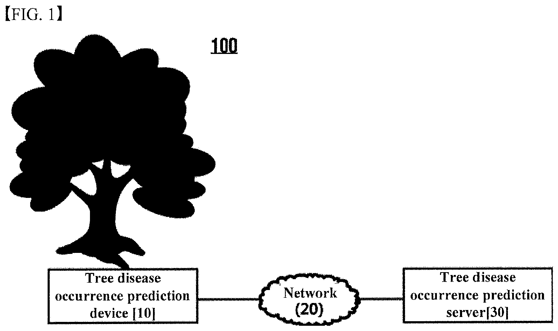

Hereinafter, a tree disease occurrence-prediction system 100 will be described with reference to FIG. 1. With reference to FIG. 1, a block diagram of the tree disease occurrence-prediction system 100 is illustrated.

With reference to FIG. 1, the tree disease-occurrence prediction system 100 may include a tree disease-occurrence prediction device 10, a network 20, and a tree disease-occurrence prediction server 30.

According to the tree disease-occurrence prediction system 100 of the present embodiment, whether a tree disease is to occur in a tree may be predicted. For this purpose, the tree disease-occurrence prediction device 10 may collect information related to the tree, which may be a judgement basis for predicting the occurrence of the tree disease. The network 20 then may allow the information collected by the tree disease-occurrence prediction device 10 to be transmitted to the tree disease-occurrence prediction server 30. The tree disease-occurrence prediction server 30 may predict a probability of the occurrence of the tree disease based on the information collected by the tree disease-occurrence prediction device 10.

In some embodiments, however, the tree disease-occurrence prediction device 10 may not only collect the information related to the tree, but may also predict the probability of the occurrence of the tree disease based on the collected information. In this case, the tree disease-occurrence prediction device 10 may also transmit the collected information and the prediction result to the tree disease-occurrence prediction server 30 via the network 20.

In this connection, a healthy tree is a normal tree. The normal tree becomes an abnormal tree when it is affected by the tree disease. The tree disease-occurrence prediction system 100 of the present embodiment may predict a probability of occurrence of the abnormal tree. The tree disease may be any one of a variety of diseases that may occur in the tree. In the present invention, the pine wilt disease caused by a pinewood nematode parasitic on Monochamus alternatus is described as the tree disease.

Hereinafter, each component included in the tree disease-occurrence prediction system 100 according to the present embodiment will be described in detail.

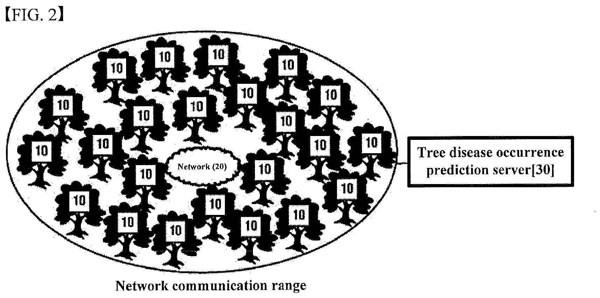

First, with reference to FIG. 2, the network 20 included in the tree disease-occurrence prediction system 100 will be described. With reference to FIG. 2, an expanded block diagram of a system for predicting occurrence of a tree disease is disclosed.

The network 20 may communication between the tree disease-occurrence prediction device 10 and the tree disease-occurrence prediction server 30 to be performed. The network 20 according to the present embodiment may support various communication schemes, for example, using Wi-Fi, Bluetooth, LTE or Low Power Wide Area (LPWA) as well as using an existing communication network.

With reference to FIG. 2, the tree disease-occurrence prediction system 100 according to the present embodiment may be applied not only to small areas where tens of trees are planted, but also to mountains where thousands or tens of thousands of trees are planted. The network 20 according to the present embodiment employs the LPWA scheme, and may transmit, a long distance, the information collected by the tree disease-occurrence prediction device 10. Therefore, the tree disease-occurrence prediction system 100 according to the present embodiment may be introduced with minimizing the number of base stations necessary for the communication. Therefore, the tree disease-occurrence prediction system 100 according to the present embodiment may be easily applied to the mountains.

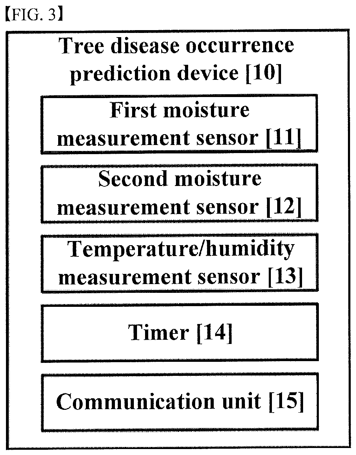

Subsequently, with reference to FIGS. 3 to 8, the tree disease-occurrence prediction device 10 included in the tree disease-occurrence prediction system 100 will be described.

### [First embodiment]

With reference to FIG. 3, the tree disease-occurrence prediction device 10 may collect the information related to the tree, which may be the judgement basis for predicting the occurrence of the tree disease, and transmit the collected information to the tree disease-occurrence prediction server 30 via the network 20. The tree disease-occurrence prediction device 10 may include first and second moisture measurement sensors 11 and 12, a temperature/humidity measurement sensor 13, a timer 14, and a communication unit 15. However, a configuration of the tree disease-occurrence prediction device 10 may be not limited thereto, and may be composed of more or fewer components than those illustrated in FIG. 3.

The first moisture measurement sensor 11 may be disposed on the tree to measure moisture information about the tree. In this connection, the moisture information may include a moisture content. Specifically, the first moisture measurement sensor 11 may measure a moisture content in the tree using a heat pulse method. In order to dispose the first moisture measurement sensor 11 in the tree, a hole may be defined in the tree in an invasive manner. The first moisture measurement sensor 11 may be disposed in the corresponding hole.

The second moisture measurement sensor 12 may be disposed in soil where the tree is located to measure moisture information about the soil, or may be disposed in soil nearby where the tree is located to measure the moisture information about the soil. In this connection, the moisture information about the soil may include a moisture content. Specifically, the second moisture measurement sensor 12 may measure a moisture content of the soil where the tree is growing. For example, the second moisture measurement sensor 12 may be inserted into the soil near a root of the tree. This may allow the second moisture measurement sensor 12 to collect the moisture information about the soil placed near the root of the tree.

The tree disease-occurrence prediction device 10 according to the present embodiment simultaneously collects not only the moisture information about the tree but also the moisture information about the soil. A reason for this is to predict whether the tree disease is to occur based on the moisture information about the soil as well as the moisture information about the tree.

Generally, in a case of the normal tree, when a moisture content of a soil increases, a moisture content of a tree growing in the soil increases after a certain period of time. In addition, when the moisture content of the soil decreases, the moisture content of the tree growing in the soil also decreases after a certain period of time. However, a change range of the water content of the tree may be relatively small compared with a change range of the water content of the soil.

In contrast, in a case of the abnormal tree, a water content of a tree may continue to decrease regardless of changes such as increasing or decreasing of a moisture content of a soil. Therefore, whether the tree disease is to occur may be predicted based on the moisture information of the tree and the moisture information of the soil. Specifically, whether the tree disease is to occur may be predicted based on whether a difference between the moisture information of the tree and the moisture information of the soil has a level equal to or greater than a predetermined level.

In some embodiments, whether the tree disease is to occur may be predicted based on a trend of change of the moisture information about the tree over time and a trend of change of the moisture information of the soil over time. This will be described below.

Further, the tree disease-occurrence prediction device 10 may be disposed for each tree. In this case, the first moisture measurement sensor 11 may be disposed on each tree, and a second moisture measurement sensor 12 may be disposed in soil near a root of each tree.

The temperature/humidity measurement sensor 13 may measure temperature and humidity information around the tree. In order to measure the temperature and humidity information, the temperature/humidity measurement sensor 13 may be attached to the tree. Thus, the tree disease-occurrence prediction system 100 according to the present embodiment may collect environment information around the tree.

The timer 14 is a device for calculating time information, and may be configured, for example, as a relay, but is not limited thereto. The tree disease-occurrence prediction device 10 may use the time information from the timer 14 to specify a time at which the information is measured by the sensors 11, 12, and 13. In addition, the tree disease-occurrence prediction device 10 may transmit the time information along with the collected information to the tree disease-occurrence prediction server 30.

The communication unit 15 may exchange the information with an external device, and may transmit the information measured by the first and second moisture measurement sensors 11 and 12 and the information calculated by the timer 14 to the tree disease-occurrence prediction server 30, for example, via the network 20.

In some embodiments, information about a location of the tree disease-occurrence prediction device 10 may be stored in the communication unit 15. Accordingly, the communication unit 15 may transmit the location information to the tree disease-occurrence prediction server 30 as well. In this connection, the location information may be information about an actual location, but is not limited thereto and may be predetermined code information.

For example, when the communication unit 15 uses the LPWA scheme, a radio wave may be transmitted over a long distance. Thus, the tree disease-occurrence prediction device 10 according to the present embodiment may be advantageous for use in areas where a large number of trees are densified such as the mountains. Furthermore, when the LPWA scheme is used, battery consumption of the communication unit 15 is not large. Therefore, the tree disease-occurrence prediction device 10 according to the present embodiment may be advantageous for use in the mountain where power supply is not easy. However, the communication scheme employed in this communication unit 15 is not limited to the LPWA scheme, and the communication unit 15 may employ various communication schemes.

In some embodiments, the communication unit 15 may transmit the information measured by the first and second moisture measurement sensors 11 and 12 and the information calculated by the timer 14 to the tree disease-occurrence prediction server 30 at a predetermined period (e.g., two hours), but is not limited thereto.

The tree disease-occurrence prediction device 10 according to some embodiments may include a battery for increasing an output of the communication unit 15 to increase a reach of the radio wave. The battery may be a 9 volt battery, but is not limited thereto.

### [Second embodiment]

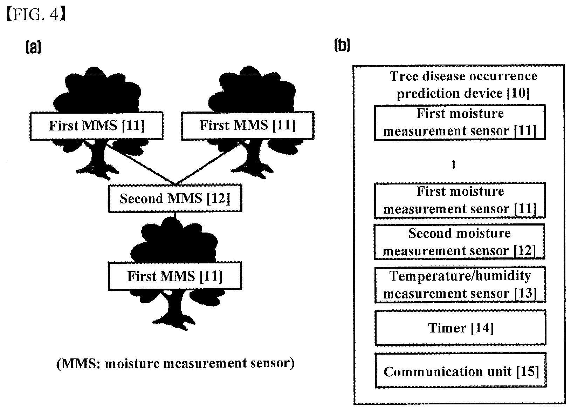

With reference to FIG. 4, the first moisture measurement sensor 11 may be disposed for each tree while the second moisture measurement sensor 12 may be disposed to be shared by the plurality of trees. In soil with a plurality of trees located adjacent to each other, a portion of the soil may have a similar characteristics as the other portion of the soil. Therefore, there is no problem that the plurality of trees share the second moisture measurement sensor 12 for measuring the moisture information about the soil.

In this case, the number of the first moisture measurement sensor 11 and the number of the second moisture measurement sensor 12 included in the tree disease-occurrence prediction device 10 according to the present embodiment may be different from each other. In addition, the number of the first moisture measurement sensor 11 may be relatively larger than the number of the second moisture measurement sensor 12.

### [Third embodiment]

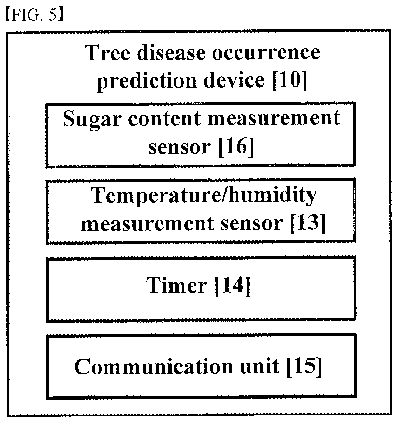

With reference to FIG. 5, the tree disease-occurrence prediction device 10 according to a third embodiment may include a sugar content measurement sensor 16 instead of the first and second moisture measurement sensors 11 and 12. The sugar content measurement sensor 16 may be disposed on the tree to measure sugar content information about the tree. For example, the sugar content information about the tree may be measured in an optical manner, in this case a near-infrared spectroscopy sensor may be used as the sugar content measurement sensor 16. For example, the sugar content measurement sensor may be used to measure changes in cellulose, glucose, or lignin in the tree.

In general, the pinewood nematode has a tendency to eat and digest the cellulose, and then make the glucose. Accordingly, a sugar content of the abnormal tree with the pine wilt disease may be higher than that of the healthy tree. Therefore, the tree disease may be more likely to occur when the sugar content of the tree is abnormally increased. For example, when the sugar content information about the tree is increased by a predetermined ratio (e.g., 5 %) or greater within a predetermined period or a predetermined time, the tree disease may be expected to occur.

Thus, the tree disease-occurrence prediction device 10 according to the present embodiment may use the sugar content measurement sensor 16 to measure the sugar content information for detecting the changes in the cellulose, the glucose or the lignin in the tree, thereby collecting information for predicting whether the tree disease is to occur.

### [Fourth embodiment]

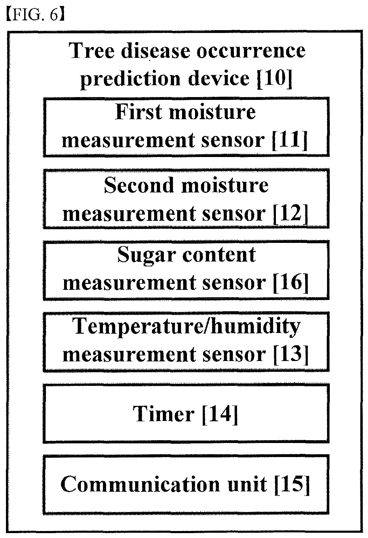

With reference to FIG. 6, the tree disease-occurrence prediction device 10 according to the fourth embodiment may include the first and second moisture measurement sensors 11 and 12 and the sugar content measurement sensor 16. Thus, the tree disease-occurrence prediction device 10 according to the present embodiment may collect various information for predicting the occurrence of the tree disease.

### [Fifth embodiment]

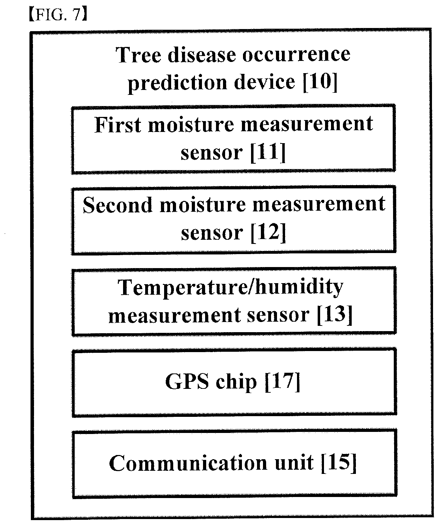

With reference to FIG. 7, the tree disease-occurrence prediction device 10 according to the fifth embodiment may include a GPS chip 17 instead of the timer 14. The GPS chip 17 may measure position information of the tree. The position information from the GPS chip 17 may be utilized to specify the tree corresponding to the position information. In addition, when the corresponding tree is the abnormal tree, the corresponding tree may be removed or treated.

### [Sixth embodiment]

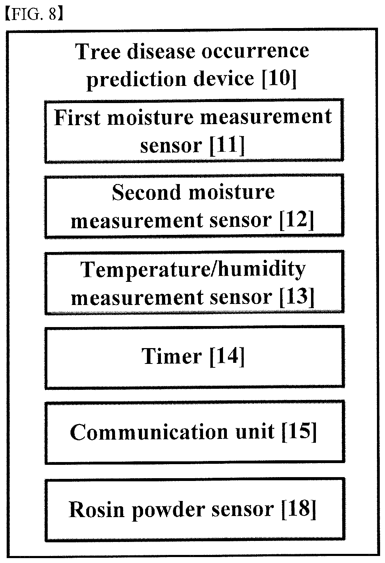

With reference to FIG. 8, the tree disease-occurrence prediction device 10 according to the sixth embodiment may further include a rosin powder sensor 18. The rosin powder sensor 18 may measure an amount of the rosin powder in the tree. In a case of the abnormal tree with the pine wilt disease, the rosin powder does not occur much as compared to the normal tree. Thus, whether the tree disease has occurred may be predicted through the amount of the rosin powder measured by the rosin powder sensor 18.

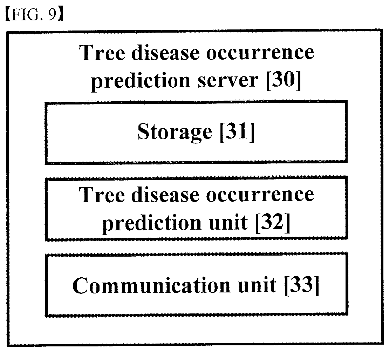

Next, with reference to FIG. 9, the tree disease-occurrence prediction server 30 included in the tree disease-occurrence prediction system 100 will be described.

With reference to FIG. 9, the tree disease-occurrence prediction server 30 may predict the probability of the occurrence of the tree disease based on the information collected by the tree disease-occurrence prediction device 10. The tree disease-occurrence prediction server 30 may include storage 31, a tree disease-occurrence prediction unit 32, and a communication unit 33. However, the tree disease-occurrence prediction server 30 may be composed of more or fewer components than those illustrated in FIG. 9.

The storage 31 may receive and store the information collected from the tree disease-occurrence prediction device 10, and may further store the information necessary for predicting whether the tree disease is to occur. For example, the storage 31 may store the moisture information about the tree measured from the first moisture measurement sensor 11 disposed on the tree, the moisture information about the soil measured from the second moisture measurement sensor 12 disposed in the soil where the tree is located, or the sugar content information about the tree measured from the sugar content measurement sensor 16, and the like, but is not limited thereto.

The tree disease-occurrence prediction unit 32 may predict whether the tree disease is to occur based on the moisture information about the tree and the moisture information about the soil. For example, the tree disease-occurrence prediction unit 32 may predict whether the tree disease is to occur based on whether the difference between the moisture information about the tree and the moisture information about the soil has a level equal to or greater than the predetermined level.

In addition, the tree disease-occurrence prediction unit 32 may predict whether the tree disease is to occur in a step manner based on a level of the difference between the moisture information about the tree and the moisture information about the soil. For example, the water content of the tree may not increase or may decrease continuously for a certain period (10-14 days) even though the moisture content of the soil increases. In addition, the difference in the moisture information between the tree and the soil may have a level of 10 % or greater, that is, even after the certain period of time, the moisture content of the tree may be higher or lower equal to or greater than 10% than the moisture content of the soil. In this case, the tree may be expected to be in an abnormal state in which the tree disease is likely to occur. Further, when the moisture content of the tree is 20% or greater higher or lower than the soil moisture content, it may be expected that the tree disease has occurred.

In some embodiments, the tree disease-occurrence prediction unit 32 may predict whether the tree disease is to occur based on the trend of change of the moisture information about the tree over time and the trend of change of the moisture information of the soil over time.

For example, the tree disease-occurrence prediction unit 32 may predict whether the tree disease is to occur based on whether the trend of change of the first moisture information is identical with the trend of change of the second moisture information. Specifically, in a case of the abnormal tree, the moisture content of the tree may continue to decrease even as the moisture content of the soil increases. Therefore, the tree disease-occurrence prediction unit 32 may evaluate that the possibility of the occurrence of tree disease is high when the trend of change of the first moisture information is not identical with the trend of change of the second moisture information.

Further, the tree disease-occurrence prediction unit 32 may predict whether the tree disease is to occur based on whether the first moisture information has a decreasing trend irrespective of the trend of change of the second moisture information. Specifically, in a case of the abnormal tree, the water content of the tree may continue to decrease regardless of the changes such as increasing or decreasing of the moisture content of the soil. Therefore, the tree disease-occurrence prediction unit 32 may evaluate that the possibility of the occurrence of tree disease is high when the first moisture information has the decreasing trend irrespective of the trend of change of the second moisture information.

The communication unit 33 may exchange the information with the external device, and receive the information collected from the tree disease-occurrence prediction device 10 via the network 20.

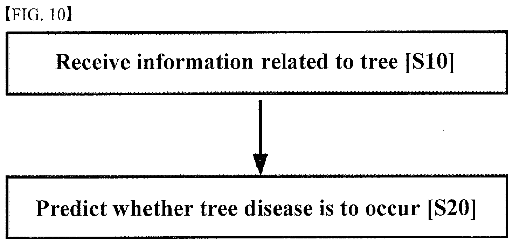

Subsequently, a flowchart of a method for predicting occurrence of a tree disease according to an embodiment of the inventive concept will be described with reference to FIG. 10. With reference to FIG. 10, a flowchart of a method for predicting occurrence of a tree disease according to an embodiment of the inventive concept is disclosed.

With reference to FIG. 10, first, the tree disease-occurrence prediction server 30 may receive the information related to the tree from the tree disease-occurrence prediction device 10 (S10).

The information related to the tree transmitted from the tree disease-occurrence prediction device 10 to the tree disease-occurrence prediction server 30 may include, for example, at least one of the moisture information measured by the first and second moisture measurement sensors 11 and 12, the temperature and humidity information around the tree measured by the temperature/humidity measurement sensor 13, the time information calculated by the timer 14, the sugar content information measured by the sugar content measurement sensor 16, the position information measured by the GPS chip 17, or the information about the amount of the rosin powder measured by the rosin powder sensor 18.

Next, the tree disease-occurrence prediction server 30 may predict whether the tree disease is to occur based on the received information S20. A description in regard to this will be omitted because it is the same as that of the tree disease-occurrence prediction unit 32.

It should be understood that the above embodiments are not limiting, but illustrative.

According to the inventive concept as defined in the appended claims, the following effects may be obtained, but the effects obtained through the inventive concept are not limited thereto.

First, according to the inventive concept, the moisture information about the tree and the soil and the sugar content information about the tree is used to predict whether the tree disease is to occur. Thus, a prediction accuracy of the tree disease may be improved.

Second, according to the inventive concept, the information measured by the sensor is transmitted and received using the low power wide area (LPWA) scheme. Thus, there is no problem to use the inventive concept in the mountain.

## Claims

1. A system for predicting occurrence of a pine wilt disease, the system comprising:
a first moisture measurement sensor (11) disposed on a tree for measuring first moisture information about the tree;
a second moisture measurement sensor (12) disposed in soil where the tree is located for measuring second moisture information about the soil;
a sugar content measurement sensor (16) disposed on the tree for measuring sugar content information about the tree;
storage (31) configured for receiving and storing:
first moisture information about the tree measured by the first moisture measurement sensor (11) disposed on the tree; and
second moisture information about soil measured by the second moisture measurement sensor (12) disposed in the soil where the tree is located; and
sugar content information about the tree measured by the sugar content measurement sensor (16) disposed on the tree; and
a tree disease-occurrence prediction unit (32) configured for predicting whether the pine wilt disease is to occur based on the first moisture information, the second moisture information, and the sugar content information,
a GPS chip for measuring position information of the tree; and
a communication unit (15) configured for transmitting the information measured from the first and second moisture measurement sensors (11,12) and the sugar content measurement sensor (16), and the position information measured by the GPS chip to an external device in a low power wide area (LPWA) manner, and
a battery for increasing an output of the communication unit (15).

2. A method for predicting occurrence of a pine wilt disease with a system according to claim 1, the method comprising:
receiving the first moisture information about a tree;
receiving the second moisture information about soil;
receiving the sugar content information about the tree; and
predicting whether the tree disease is to occur based on the first moisture information, the second moisture information and the sugar content information.

3. The method of claim 2, wherein the tree disease-occurrence prediction unit (32) predicts whether the pine wilt disease is to occur based on whether the sugar content information has a level equal to or greater than a predetermined level.

4. The method of claim 2, wherein the tree disease-occurrence prediction unit (32) predicts whether the tree disease is to occur based on whether a difference between the first moisture information and the second moisture information has a level equal to or greater than a predetermined level.

5. The method of claim 2, wherein the tree disease-occurrence prediction unit (32) predicts whether the tree disease is to occur based on a trend of change of the first moisture information over time and a trend of change of the second moisture information over time.

6. The method of claim 5, wherein the tree disease-occurrence prediction unit (32) predicts whether the tree disease is to occur based on whether the trend of change of the first moisture information is identical with the trend of change of the second moisture information.

7. The method of claim 5, wherein the tree disease-occurrence prediction unit (32) predicts whether the tree disease is to occur based on whether the first moisture information has a decreasing trend irrespective of the trend of change of the second moisture information.

## Patentansprüche

1. System zum Vorhersagen eines Auftretens einer Kiefernwelke-Krankheit, wobei das System umfasst:
einen ersten Feuchtigkeitsmesssensor (11), der an einem Baum angeordnet ist, um eine erste Feuchtigkeitsinformation über den Baum zu messen;
einen zweiten Feuchtigkeitsmesssensor (12), der im Boden angeordnet ist, wo sich der Baum befindet, um eine zweite Feuchtigkeitsinformation über den Boden zu messen;
einen Zuckergehaltsmesssensor (16), der an dem Baum angeordnet ist, um eine Zuckergehaltsinformation über den Baum zu messen;
eine Speicherung (31), die dazu konfiguriert ist, zu empfangen und zu speichern:
eine erste Feuchtigkeitsinformation über den Baum, die durch den ersten Feuchtigkeitsmesssensor (11) gemessen wurde, der an dem Baum angeordnet ist; und
eine zweite Feuchtigkeitsinformation über den Boden, die durch den zweiten Feuchtigkeitsmesssensor (12) gemessen wurde, der im Boden angeordnet ist, wo sich der Baum befindet; und
eine Zuckergehaltsinformation über den Baum, die durch den Zuckergehaltsmesssensor (16) gemessen wurde, der an dem Baum angeordnet ist; und
eine Einheit zur Vorhersage eines Auftretens einer Baumkrankheit (32), die dazu konfiguriert ist, basierend auf der ersten Feuchtigkeitsinformation, der zweiten Feuchtigkeitsinformation und der Zuckergehaltsinformation vorherzusagen, ob die Kiefernwelke-Krankheit auftreten wird,
einen GPS-Chip zum Messen einer Positionsinformation des Baums;
eine Kommunikationseinheit (15), die dazu konfiguriert ist, die Information, die von dem ersten und dem zweiten Feuchtigkeitsmesssensor (11, 12) und dem Zuckergehaltsmesssensor (16) gemessen wurde, und die Positionsinformation, die durch den GPS-Chip gemessen wurde, in einer Niedrigenergieweitbereichs (*Low Power Wide Area; LPWA*)-Weise zu einer externen Vorrichtung zu übertragen; und
eine Batterie zum Steigern eines Outputs der Kommunikationseinheit (15).

2. Verfahren zum Vorhersagen eines Auftretens einer Kiefernwelke-Krankheit mit einem System nach Anspruch 1, wobei das Verfahren umfasst:
Empfangen der ersten Feuchtigkeitsinformation über einen Baum;
Empfangen der zweiten Feuchtigkeitsinformation über einen Boden;
Empfangen der Zuckergehaltsinformation über den Baum; und
Vorhersagen, ob die Baumkrankheit auftreten wird, basierend auf der ersten Feuchtigkeitsinformation, der zweiten Feuchtigkeitsinformation und der Zuckergehaltsinformation.

3. Verfahren nach Anspruch 2, wobei die Einheit zur Vorhersage eines Auftretens einer Baumkrankheit (32) basierend darauf, ob die Zuckergehaltsinformation ein Niveau aufweist, das gleich einem oder größer als ein vorgegebene Niveau ist, vorhersagt, ob die Kiefernwelke-Krankheit auftreten wird.

4. Verfahren nach Anspruch 2, wobei die Einheit zur Vorhersage eines Auftretens einer Baumkrankheit (32) basierend darauf, ob ein Unterschied zwischen der ersten Feuchtigkeitsinformation und der zweiten Feuchtigkeitsinformation ein Niveau aufweist, das gleich einem oder größer als ein vorgegebenes Niveau ist, vorhersagt, ob die Baumkrankheit auftreten wird.

5. Verfahren nach Anspruch 2, wobei die Einheit zur Vorhersage eines Auftretens einer Baumkrankheit (32) basierend auf einem Änderungstrend der ersten Feuchtigkeitsinformation über die Zeit und einem Änderungstrend der zweiten Feuchtigkeitsinformation über die Zeit vorhersagt, ob die Baumkrankheit auftreten wird.

6. Verfahren nach Anspruch 5, wobei die Einheit zur Vorhersage eines Auftretens einer Baumkrankheit (32) basierend darauf, ob der Änderungstrend der ersten Feuchtigkeitsinformation identisch zu dem Änderungstrend der zweiten Feuchtigkeitsinformation ist, vorhersagt, ob die Baumkrankheit auftreten wird.

7. Verfahren nach Anspruch 5, wobei die Einheit zur Vorhersage eines Auftretens einer Baumkrankheit (32) basierend darauf, ob die erste Feuchtigkeitsinformation einen abnehmenden Trend aufweist, ungeachtet des Änderungstrends der zweiten Feuchtigkeitsinformation vorhersagt, ob die Baumkrankheit auftreten wird.

## Revendications

1. Système de prédiction de l'apparition d'une maladie de flétrissement du pin, le système comprenant :
un premier détecteur de mesure d'humidité (11) placé sur un arbre afin de mesurer une première information d'humidité concernant l'arbre,
un deuxième détecteur de mesure d'humidité (12) placé dans un sol où l'arbre est situé afin de mesurer une deuxième information d'humidité concernant le sol,
un détecteur de mesure du contenu en sucre (16) placé sur l'arbre afin de mesurer une information de contenu en sucre concernant l'arbre,
un stockage (31) configuré pour recevoir et stocker :
la première information d'humidité concernant l'arbre mesurée par le premier détecteur de mesure d'humidité (11) placé sur l'arbre et
la deuxième information d'humidité concernant le sol mesuré par le deuxième détecteur de mesure de l'humidité (12) placé dans un sol où l'arbre est situé et
l'information du contenu en sucre concernant l'arbre mesuré par le détecteur de mesure de contenu en sucre (16) placé sur l'arbre et
une unité de prédiction de l'apparition d'une maladie de l'arbre (32) configurée pour prédire si la maladie de flétrissement du pin est sur le point d'apparaître sur la base de la première information d'humidité, de la deuxième information d'humidité et l'information de contenu en sucre,
une puce GPS pour mesurer l'information de position de l'arbre et
une unité de communication (15) configurée pour transmettre l'information mesurée à partir des premiers et deuxièmes détecteurs de mesure de l'humidité (11, 12) et le détecteur de mesure du contenu en sucre (16) et l'information de position mesurée par la puce GPS à un dispositif externe d'une manière en bande ultra-étroite étendue (LPWA) et
une batterie destinée à augmenter une émission de l'unité de communication (15).

2. Procédé de prédiction de l'apparition d'une maladie de flétrissement du pin avec un système selon la revendication 1, le procédé comprenant :
la réception de la première information d'humidité concernant un arbre,
la réception de la deuxième information d'humidité concernant le sol,
la réception de l'information de contenu en sucre concernant l'arbre et
la prévision de la possibilité d'apparition de la maladie des arbres sur la base de la première information d'humidité, la deuxième information d'humidité et l'information du contenu en sucre.

3. Procédé selon la revendication 2, **caractérisé en ce que** l'unité de prédiction de l'apparition d'une maladie de l'arbre (32) prédit si la maladie de flétrissement du pin est sur le point d'apparaître sur la base de savoir si l'information du contenu en sucre présente un niveau égal ou supérieur à un niveau prédéterminé.

4. Procédé selon la revendication 2, **caractérisé en ce que** l'unité de prédiction de l'apparition de la maladie de l'arbre (32) prédit si la maladie de l'arbre est sur le point d'apparaître sur la base d'une différence entre la première information d'humidité et la deuxième information d'humidité étant d'un niveau égal ou supérieur à un niveau prédéterminé.

5. Procédé selon la revendication 2, **caractérisé en ce que** l'unité de prédiction de l'apparition de la maladie de l'arbre (32) prédit si la maladie de l'arbre est sur le point d'arriver sur la base d'une tendance du changement de la première information d'humidité au cours du temps et d'une tendance du changement de la deuxième information d'humidité au cours du temps.

6. Procédé selon la revendication 5, **caractérisé en ce que** l'unité de prédiction de l'apparition de la maladie de l'arbre (32) prédit si la maladie de l'arbre est sur le point d'apparaître sur la base de savoir si la tendance du changement de la première information d'humidité est identique à la tendance du changement de la deuxième information d'humidité.

7. Procédé selon la revendication 5, **caractérisé en ce que** l'unité de prédiction de l'apparition de la maladie de l'arbre (32) prédit si la maladie de l'arbre est sur le point d'apparaître sur la base de savoir si la première information d'humidité présente une tendance décroissante indépendamment de la tendance du changement de la deuxième information d'humidité.
